# EUROPEAN PATENT SPECIFICATION

(11) **EP 2 170 797 B1**
(45) Date of publication and mention of the grant of the patent: **09.03.2011**
(21) Application number: 08774907.3
(22) Date of filing: 09.07.2008
(51) Int. Cl.: C07C 51/377, C07C 53/126

(54) **PROCESS FOR CONVERTING LEVULINIC ACID INTO PENTANOIC ACID**
VERFAHREN ZUM UMWANDELN VON LEVULINSÄURE IN PENTANSÄURE
PROCÉDÉ DE CONVERSION DE L'ACIDE LÉVULINIQUE EN ACIDE PENTANOÏQUE

(30) Priority: 12.07.2007 EP 07112330
(43) Date of publication of application: 07.04.2010
(73) Proprietor: Shell Internationale Research Maatschappij B.V., 2596 HR The Hague (NL)
(72) Inventor: LANGE, Jean-Paul, NL-1031 CM Amsterdam (NL)
(74) Representative: Matthezing, Robert Maarten
(86) International application number: PCT/EP2008/058901
(87) International publication number: WO 2009/007391

(56) References cited:
- WO-A-2006/067171
- DATABASE CA [Online] CHEMICAL ABSTRACTS SERVICE, COLUMBUS, OHIO, US; 1955, HAYASHI, IZUMI ET AL: "Levulinic acid and its derivatives. II. Preparation and reduction of .gamma.-valerolactone by catalytic hydrogenation" XP002463295 retrieved from STN Database accession no. 1955:60246 & NIPPON KAGAKU KAISHI (1921-47) , IND. CHEM. SECT. 57, 67-9 CODEN: NIKWAB; ISSN: 0369-4208, 1954,

## Description

### Field of the invention

The present invention provides a process for converting levulinic acid into pentanoic acid.

### Background of the invention

It is known that levulinic acid or its esters can be converted into gamma valerolactone by catalytic hydrogenation. The conversion may proceed via hydrogenation to 4-hydroxy pentanoic acid followed by (trans)esterification to gamma valerolactone or via (trans)esterification of the enol form of levulinic acid to angelica lactone followed by hydrogenation to gamma valerolactone. The gamma valerolactone thus-formed may be further converted into pentanoic acid.

In WO2006/067171 is disclosed a process for the hydrogenation of levulinic acid via gamma valerolactone into pentanoic acid in a single reactor containing a heterogeneous bi-functional catalyst, i.e. a strongly acidic heterogeneous catalyst having a hydrogenating component.

If levulinic acid is used as reactant in the process of WO2006/067171, catalyst deactivation might occur by leaching due to the presence of acid reactant and acid reaction product, by poisoning due to the presence of reaction water, and/or by fouling due to oligomerisation or polymerisation of unsaturated intermediates such as angelica-lactone and pentenoic acid in the presence of an acid catalyst.

Since the hydrogenation of levulinic acid into pentanoic acid is highly exothermic, careful temperature control is very important to prevent undesired catalyst deactivation or side-reactions.

### Summary of the invention

It has now been found that catalyst deactivation and tar formation can be reduced in the catalytic hydrogenation of levulinic acid into pentanoic acid over a heterogeneous bi-functional catalyst, or over a non-acidic heterogeneous hydrogenation catalyst in the presence of an homogeneous acid, by carrying out the reaction in two catalytic zones in series, wherein the first zone is operated at a lower temperature than the second zone. The two catalytic zones are preferably the upstream and the downstream part of a single catalyst bed.

Accordingly, the invention provides a process for converting levulinic acid into pentanoic acid, comprising the following steps:
(a) supplying hydrogen and a feedstock comprising levulinic acid to a first catalytic zone comprising a strongly acidic catalyst and a hydrogenation metal;
(b) converting, in the first catalytic zone, the levulinic acid at a temperature in the range of from 100 to 250 °C into gamma valerolactone to obtain a first effluent comprising gamma valerolactone;
(c) supplying at least part of the first effluent to a second catalytic zone comprising a strongly acidic catalyst and a hydrogenation metal; and
(d) converting, in the second catalytic zone, gamma valerolactone into pentanoic acid at a temperature in the range of from 200 to 350 °C to obtain a second effluent comprising pentanoic acid,
wherein the conversion temperature in the first catalytic zone is lower than the conversion temperature in the second catalytic zone, and wherein the acidic catalyst and the hydrogenation metal in the first catalytic zone has the same composition as the acidic catalyst and the hydrogenation metal in the second catalytic zone.

In the first catalytic zone, levulinic acid is converted into gamma valerolactone. In the second catalytic zone, the gamma valerolactone is further converted into pentanoic acid. An advantage of the process according to the invention as compared to a process as disclosed in WO2006/067171, i.e. a process using a single bed of bifunctional catalyst without a temperature profile over the bed, is that tar formation is reduced since the temperature is relatively low in the part of the catalytic zone where tar precursors are present. In the process according to the invention, the concentration of levulinic acid in the higher temperature zone, i.e. the second catalytic zone, is low. Preferably the process is operated such that the concentration of levulinic acid in the first effluent is at most 3 wt%, more preferably at most 1 wt%.

Preferably, the process is operated such that in the second catalytic zone only part of the gamma valerolactone is converted into pentanoic acid. The second effluent can then be separated into a stream enriched in gamma valerolactone and a stream enriched in pentanoic acid in order to recycle the stream enriched in gamma valerolactone to the first catalytic zone. An advantage of such recycle is that the heat released by the exothermic hydrogenation reaction can be better accommodated. Another advantage of such recycle is that there is less tar formation, since the precursors for tar formation, in particular angelica-lactone and pentenoic acid, are diluted by the gamma valerolactone recycle. Moreover, recycling of gamma valerolactone in combination with cooling of the recycle stream will provide for additional heat removal.

A further advantage of such recycle is that catalyst deactivation due to leaching of acid from the catalyst is reduced, since the concentration of acid reactant, i.e. levulinic acid, and acid product, i.e. pentanoic acid, is reduced.

### Brief description of the drawing

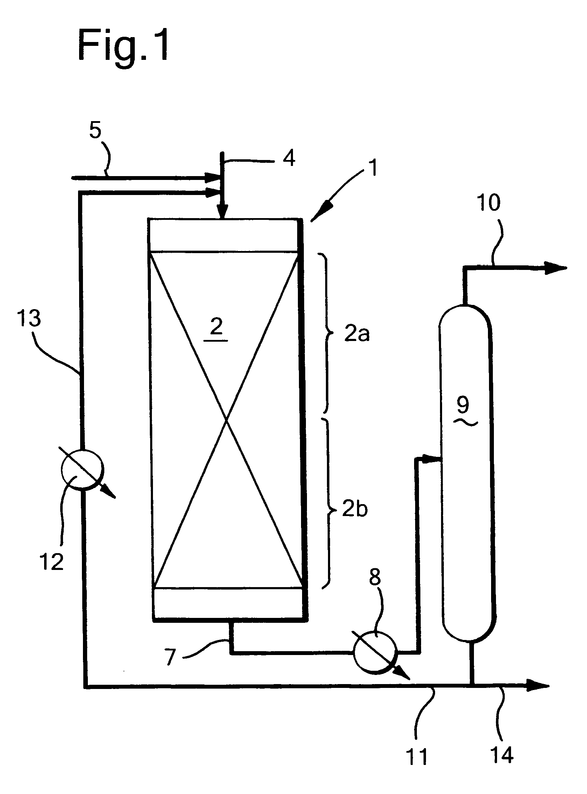

Figure 1 shows an embodiment of the invention wherein the hydrogenation is carried out in a single adiabatically-operated catalyst bed with a cooled recycle of gamma valerolactone.

### Detailed description of the invention

In the process according to the invention, hydrogen and a feedstock comprising levulinic acid are supplied to a first catalytic zone for conversion of the levulinic acid into gamma valerolactone at a temperature in the range of from 100 to 250 °C, preferably of from 125 to 200 °C, to obtain a first effluent comprising gamma valerolactone. At least part of the first effluent is supplied to a second catalytic zone operating at a temperature in the range of from 200 to 350 °C, preferably of from 250 to 300 °C, for conversion of gamma valerolactone into pentanoic acid. A second effluent comprising pentanoic acid is obtained in the second catalytic zone.

The conversion temperature in the first catalytic zone is lower than the conversion temperature in the second catalytic zone. There may be a temperature profile over each or one of the catalytic zones. In case of such profile, reference to the conversion temperature in a zone is to the weight averaged bed temperature. Preferably, the conversion temperature in the first zone is in the range of from 30 to 150 °C lower than the conversion temperature in the second zone.

Both zones comprise a strongly acidic and a hydrogenating catalytic function, i.e. a strongly acidic catalyst and at least one hydrogenation metal. The catalytic functions in each zone are of the same composition. The strongly acidic catalyst and the hydrogenation metal may either be in the form of a bi-functional heterogeneous catalyst, i.e. a solid catalyst having both an acidic and a hydrogenation function, or in the form of a non-acidic solid hydrogenation catalyst and a liquid acidic catalyst.

Preferably, the entire first effluent is supplied to the second catalytic zone. Alternatively, part of the first effluent is supplied to the second catalytic zone and part of the first effluent is recycled to the first catalytic zone.

The feedstock supplied to the first catalytic zone preferably comprises at least 50 wt% levulinic acid, more preferably at least 70 wt%, even more preferably at least 90 wt%.

Hydrogen may be supplied to the first catalytic zone as pure hydrogen or as a hydrogen-containing gas. Hydrogen-containing gases suitable for hydrogenation reactions are well-known in the art.

The hydrogen to levulinic acid molar ratio supplied to the first catalytic zone is typically in the range of from 0.1 to 20. Preferably, an amount of hydrogen in excess of the stoichiometric amount is used in order to minimise the amount of the polymerising intermediate product alpha-angelicalactone. Therefore, the hydrogen to levulinic acid molar ratio supplied to the first catalytic zone is preferably in the range of from 1.1 to 5.0.

Also for step (d), i.e. the conversion of gamma valerolactone into pentanoic acid, hydrogen is needed. Typically, the amount of hydrogen present in the first effluent that is supplied to the second catalytic zone will contain sufficient hydrogen for step (d). Additional hydrogen may, however, be supplied to the second catalytic zone.

The hydrogen pressure in both zones is preferably in the range of from 1 to 150 bar (absolute), more preferably of from 10 to 50 bar (absolute).

In the first catalytic zone, the feedstock and the first effluent are in the liquid phase; the hydrogen supplied to the first zone is in the gas phase; and the catalyst is a bi-functional solid catalyst or a combination of solid and liquid catalyst. Thus, the conversion reaction in the first catalytic zone is a gas/liquid/solid reaction. In the second catalytic zone, the feed, i.e. the first effluent may be in the liquid or gas phase. Thus, the conversion reaction in the second catalytic zone is a gas/liquid/solid reaction or a gas/gas/solid reaction.

The first and the second catalytic zone may be contained in a single reactor vessel or in separate reactor vessels in series, preferably in a single reactor vessel. If contained in a single vessel, the two zones may be two different catalytic zones or may together form a single catalyst bed. Preferably, the two zones are the upstream and the downstream part of a single catalyst bed in such way that the two zones together form the entire catalyst bed. Reference herein to upstream and downstream is with respect to the flow of the feedstock.

Preferably, the volume of the first catalytic zone is in the range of from 20 to 80 vol% of the combined volume of the first and the second catalytic zone, more preferably in the range of from 30 to 60 vol%.

Preferably, the first and the second catalytic zones are in the form of a fixed arrangement of catalyst and steps (b) and (d) are operated in trickle flow. Alternatively, each or one of the steps are operated in a slurry bubble column or a fluidised bed. It will be appreciated that for two different reaction regimes for the two steps, e.g. a slurry regime followed by trickle flow, the process will typically be carried out in two different reactor vessels in series.

In order to achieve the desired conversion temperatures in the first and the second catalytic zones, each of the catalytic zones may be operated isothermally, adiabatically or with a otherwise controlled temperature gradient. Internal cooling will typically be applied in case of an isothermally operated catalytic zone. Preferably, both catalytic zones are operated adiabatically, preferably in combination with a cooled recycle stream.

The conversion of levulinic acid into gamma valerolactone in the first catalytic zone is preferably at least 80%, more preferably at least 90%, even more preferably at least 95%. It is preferred that the concentration of levulinic acid in the first effluent is less than 3 wt%, more preferably less than 1 wt%.

Preferably, the gamma valerolactone conversion in the second catalytic zone is not complete, thus obtaining a second effluent comprising gamma valerolactone, and part of the gamma valerolactone in the second effluent is recycled to the first catalytic zone. In this way the tar precursors in the first catalytic zone are diluted and the heat released by the exothermic reaction can be removed by cooling the recycle stream. Moreover, the concentration of acids in the first catalytic zone is reduced, therewith reducing the risk of leaching of the catalyst.

In order to provide for sufficient gamma valerolactone recycle, the conversion of gamma valerolactone into pentanoic acid in the second catalytic zone is preferably at most 70 wt%, more preferably in the range of from 20 to 50 wt%.

In case of a gamma valerolactone recycle, the second effluent is separated into a stream enriched in gamma valerolactone and a stream enriched in pentanoic acid. This may be done by any suitable separation techniques known in the art, for example by distillation. The stream enriched in gamma valerolactone is recycled to the first catalytic zone. Preferably, the stream enriched in gamma valerolactone is cooled before being recycled to the first catalytic zone, more preferably cooled to a temperature in the range of from 20 to 200 °C, even more preferably of from 40 to 100 °C.

The stream enriched in pentanoic acid typically comprises pentanoic acid, reaction water, unreacted hydrogen and, optionally, other reaction products such as methyltetrahydrofuran, pentanol and pentanediol, and optionally unconverted levulinic acid. The hydrogen is preferably separated from the stream enriched in pentanoic acid and recycled to the first and/or second catalytic zone. The pentanoic acid is preferably recovered as product from the stream enriched in pentanoic acid.

Preferably, the rate of feedstock supply and the rate of recycle to the first catalytic zone are such that the molar ratio of levulinic acid-to-gamma valerolactone supplied to the hydrogenating reactor is in the range of from 0.05 to 5.0, more preferably of from 0.1 to 2.0, even more preferably of from 0.2 to 0.5.

The strongly acidic catalyst and the hydrogenation metal are preferably combined in a bi-functional catalyst, i.e. an heterogeneous strongly acidic catalyst having a hydrogenation metal. In case of a heterogeneous strongly acidic catalyst having a hydrogenation metal, the catalyst preferably comprises an acidic zeolite, more preferably acidic zeolite beta or acidic ZSM-5, supporting at least one hydrogenation metal. Alternatively, such catalysts may comprise an acidic mixed oxide, sulphonated carbon, or temperature-resistant sulphonated resins.

Alternatively, the strongly acidic catalyst is an homogeneous strongly acidic catalyst, for example a mineral acid or heteropolyacid such as tungstenphosphate or tungstensilicate, and the hydrogenation metal is supported on a solid non-acidic catalyst support, for example silica, titania or zirconia. Preferably, the liquid strongly acidic catalyst is a mineral acid, more preferably sulphuric acid or phosphoric acid, even more preferably sulphuric acid.

In case an homogeneous strongly acidic catalyst is used, the liquid strongly acidic catalyst is preferably recycled to the first catalytic zone after separation from the second effluent. In case of a gamma valerolactone recycle, the liquid acidic catalyst is recycled to the first catalytic zone with the gamma valerolactone in the gamma valerolactone enriched stream.

An advantage of using a liquid strongly acidic catalyst in combination with a hydrogenation metal on a solid non-acidic support is that no strongly acidic catalyst support is needed, such as for example an acidic zeolite, and that leaching of such support due to the presence of acid reactant (levulinic acid) or reaction product (pentanoic acid) is avoided.

The hydrogenation metal in the bi-functional catalyst or supported on the solid non-acidic catalyst support is preferably a metal of any one of column 7 to 11 of the Periodic table of Elements, more preferably Ru, Rh, Pt, Pd, Ir and/or Au.

### Detailed description of the drawing

In Figure 1 is shown a reactor 1 comprising a single catalyst bed (2). Catalyst bed 2 comprises an acidic heterogeneous catalyst with a hydrogenation metal. Catalyst bed has two catalytic zones 2a and 2b.

A feedstock comprising at least 90 wt% levulinic acid and hydrogen are supplied to reactor 1 via lines 4 and 5, respectively. In catalytic zone 2a, the levulinic acid is converted into gamma valerolactone. The entire effluent of the first catalytic zone flows to second catalytic zone 2b, where part of the gamma valerolactone is converted into pentanoic acid. The effluent of the second catalytic zone is withdrawn from reactor 1 via line 7, cooled in cooler 8, and supplied to distillation column 9 for separation in a top stream comprising hydrogen, water and pentanoic acid and a bottoms stream mainly comprising gamma valerolactone. The top stream is withdrawn from column 9 via line 10 and the bottoms stream is withdrawn via line 11, cooled in cooler 12 and recycled to reactor 1 via line 13. Part of the bottoms stream may be purged via line 14.

Reactor 1 is adiabatically operated. The conversion temperature in the first catalytic zone 2a is kept lower than the conversion temperature in the second catalytic zone 2b by the use of a cooled gamma valerolactone recycle.

### Examples

The invention is now further illustrated by means of the following non-limiting example.

### EXAMPLE 1 (according to the invention)

A reactor tube with an internal diameter of 15 mm was loaded with a fixed bed of 20.7 grams of catalyst particles (cylindrical extrudates with a diameter of 1.6 mm) diluted with 23 grams silicon carbide particles. The catalyst contained 0.7 wt% Pt on an acidic carrier of 25 wt% ZSM-5 and 75 wt% silica binder. The catalyst bed had a length of 32 cm.

The reactor tube was then placed in an oven and the catalyst was reduced for 8 hours at 300 °C under a hydrogen flow of 30 litres (STP) per hour, pressured to a pressure of 10 bar (absolute). The reactor was then heated such that a linear temperature gradient from 125 °C at the top of the catalyst bed to 275 °C at 18 cm from the top was maintained and the temperature in the lower part of the catalyst bed (18 to 32 cm from top) was maintained at 275 °C.

In order to simulate a gamma valerolactone recycle, a mixture of levulinic acid and gamma valerolactone was supplied to the top of the catalyst bed at a weight hourly space velocity of 0.5 gram (levulinic acid and gamma valerolactone) per gram catalyst per hour. Pure hydrogen was supplied to the top of the reactor at a flow rate of 20 litres (STP) per hour. The hydrogen pressure was 10 bar (absolute). The molar levulinic acid-to gamma valerolactone ratio was varied in time. The liquid product (second effluent) was analysed by means of gas/liquid chromatography.

In the table, the gamma valerolactone conversion and the selectivity for pentanoic acid (% moles based on the moles of levulinic acid and gamma valerolactone entering the reactor) at different times on stream (TOS) are given. The conversion of levulinic acid was complete, since no levulinic acid was detected in the effluent of the reactor.

**Table Results of EXAMPLE 1**

| TOS (h) | molar ratio LA: GVL | GVL conversion (mole%) | selectivity (mole%) |
|---|---|---|---|
| 0-330 | 1:3.3 | 90 | 74 |
| 325 | 1:3.3 | 77 | 79 |
| 330 | 1:1 | 73 | 78 |
| 495 | 1:1 | 45 | 74 |
| 500 | 1:3 | 42 | 75 |
| 640 | 1:3 | 38 | 72 |
| 645 | 1:1 | 29 | 71 |
| 700 | 1:1 | 19 | 57 |

### EXAMPLE 2 (comparison)

The reactor as described in EXAMPLE 1 was now operated isothermally at 275 °C and a mixture of levulinic acid and gamma valerolactone in a molar ratio of 1:4.6 was supplied to the top of the catalyst bed. All other conditions were as described in EXAMPLE 1. After 150 hours on stream, the experiment was stopped due to severe plugging of the reactor.

## Claims

1. A process for converting levulinic acid into pentanoic acid, comprising the following steps:
(a) supplying hydrogen and a feedstock comprising levulinic acid to a first catalytic zone comprising a strongly acidic catalyst and a hydrogenation metal;
(b) converting, in the first catalytic zone, the levulinic acid at a temperature in the range of from 100 to 250 °C into gamma valerolactone to obtain a first effluent comprising gamma valerolactone;
(c) supplying at least part of the first effluent to a second catalytic zone comprising a strongly acidic catalyst and a hydrogenation metal; and
(d) converting, in the second catalytic zone, gamma valerolactone into pentanoic acid at a temperature in the range of from 200 to 350 °C to obtain a second effluent comprising pentanoic acid,
wherein the conversion temperature in the first catalytic zone is lower than the conversion temperature in the second catalytic zone, and wherein the acidic catalyst and the hydrogenation metal in the first catalytic zone has the same composition as the acidic catalyst and the hydrogenation metal in the second catalytic zone.

2. A process according to claim 1, wherein the conversion temperature in the first catalytic zone is in the range of from 125 to 200 °C.

3. A process according to claim 1 or 2, wherein the conversion temperature in the second catalytic zone is in the range of from 250 to 300 °C.

4. A process according to any one of the preceding claims, wherein the conversion temperature in the first catalytic zone is in the range of from 30 to 150 °C lower than the conversion temperature in the second catalytic zone.

5. A process according to any one of the preceding claims, wherein the entire first effluent is supplied to the second catalytic zone.

6. A process according to any one of the preceding claims, wherein the first and the second catalytic zone are contained in a single reactor vessel.

7. A process according to claim 6, wherein the first and the second catalytic zone are the upstream and the downstream part, respectively, of a single catalyst bed.

8. A process according to any one of the preceding claims, wherein the volume of the first catalytic zone is in the range of from 20 to 80 vol% of the combined volume of the first and the second catalytic zone, preferably in the range of from 30 to 60 vol%.

9. A process according to any one of the preceding claims, wherein the second effluent further comprises gamma valerolactone, the process further comprising the following steps:
(e) separating the second effluent into a stream enriched in gamma valerolactone and a stream enriched in pentanoic acid; and
(f) recycling the stream enriched in gamma valerolactone to the first catalytic zone.

10. A process according to claim 9, wherein the molar ratio of levulinic acid in the feedstock and gamma valerolactone recycled to the first reaction zone is in the range of from 0.05 to 5.0, preferably of from 0.1 to 2.0, more preferably of from 0.2 to 0.5.

11. A process according to claim 9 or 10, wherein the stream enriched in gamma valerolactone is cooled before being recycled to the first reaction zone, preferably cooled to a temperature in the range of from 20 to 200 °C, more preferably of from 40 to 100 °C.

12. A process according to any one of the preceding claims, wherein the strongly acidic catalyst and the hydrogenation metal are combined in a heterogeneous strongly acidic catalyst having a hydrogenation metal.

13. A process according to any one of claims 1 to 11, wherein the strongly acidic catalyst is a liquid strongly acidic catalyst and the hydrogenation metal is supported on a solid non-acidic catalyst support.

14. A process according to any one of the preceding claims, the process further comprising the following step:
(g) recovering pentanoic acid as product from the stream enriched in pentanoic acid.

## Patentansprüche

1. Verfahren zur Umwandlung von Levulinsäure in Pentansäure, umfassend die folgenden Schritte:
(a) Zuführen von Wasserstoff und eines Levulinsäure umfassenden Ausgangsmaterials zu einer ersten katalytischen Zone, die einen stark sauren Katalysator und ein Hydriermetall aufweist;
(b) Umwandeln der Levulinsäure in der ersten katalytischen Zone bei einer Temperatur im Bereich von 100 bis 250°C in gamma-Valerolacton, um einen ersten, gamma-Valerolacton umfassenden Abfluss zu erhalten;
(c) Zuführen von mindestens einem Teil des ersten Abflusses zu einer zweiten katalytischen Zone, die einen stark sauren Katalysator und ein Hydriermetall umfasst; und
(d) Umwandeln von gamma-Valerolacton in Pentansäure in der zweiten katalytischen Zone bei einer Temperatur im Bereich von 200 bis 350°C, um einen zweiten, Pentansäure umfassenden Abfluss zu erhalten,
wobei die Umwandlungstemperatur in der ersten katalytischen Zone niedriger als die Umwandlungstemperatur in der zweiten katalytischen Zone ist und wobei der saure Katalysator und das Hydriermetall in der ersten katalytischen Zone die gleiche Zusammensetzung aufweisen wie der saure Katalysator und das Hydriermetall in der zweiten katalytischen Zone.

2. Verfahren nach Anspruch 1, wobei die Umwandlungstemperatur in der ersten katalytischen Zone im Bereich von 125 bis 200°C liegt.

3. Verfahren nach Anspruch 1 oder 2, wobei die Umwandlungstemperatur in der zweiten katalytischen Zone im Bereich von 250 bis 300°C liegt.

4. Verfahren nach einem der vorhergehenden Ansprüche, wobei die Umwandlungstemperatur in der ersten katalytischen Zone um einen Bereich von 30 bis 150°C niedriger als die Umwandlungstemperatur in der zweiten katalytischen Zone ist.

5. Verfahren nach einem der vorhergehenden Ansprüche, wobei der gesamte erste Abfluss der zweiten katalytischen Zone zugeführt wird.

6. Verfahren nach einem der vorhergehenden Ansprüche, wobei die erste und die zweite katalytische Zone in einem einzigen Reaktorbehälter enthalten sind.

7. Verfahren nach Anspruch 6, wobei die erste und die zweite katalytische Zone der stromaufwärtige bzw. der stromabwärtige Teil eines einzigen Katalysatorbetts sind.

8. Verfahren nach einem der vorhergehenden Ansprüche, wobei das Volumen der ersten katalytischen Zone im Bereich von 20 bis 80 Vol.-% des kombinierten Volumens der ersten und der zweiten katalytischen Zone liegt, vorzugsweise im Bereich von 30 bis 60 Vol.-%.

9. Verfahren nach einem der vorhergehenden Ansprüche, wobei der zweite Abfluss weiter gamma-Valerolacton aufweist und das Verfahren weiter die folgenden Schritte umfasst:
(e) Trennen des zweiten Abflusses in einen mit gamma-Valerolacton angereicherten Strom und einen mit Pentansäure angereicherten Strom; und
(f) Rückführen des mit gamma-Valerolacton angereicherten Stroms zur ersten katalytischen Zone.

10. Verfahren nach Anspruch 9, wobei das Molverhältnis von Levulinsäure im Ausgangsmaterial und gamma-Valerolacton, das zur ersten Reaktionszone rückgeführt wird, im Bereich von 0,05 bis 5,0, vorzugsweise von 0,1 bis 2,0, und besonders bevorzugt von 0,2 bis 0,5, liegt.

11. Verfahren nach Anspruch 9 oder 10, wobei der mit gamma-Valerolacton angereicherte Strom vor seiner Rückführung zur ersten Reaktionszone gekühlt wird, vorzugsweise auf eine Temperatur im Bereich von 20 bis 200°C, besonders bevorzugt von 40 bis 100°C, gekühlt wird.

12. Verfahren nach einem der vorhergehenden Ansprüche, wobei der stark saure Katalysator und das Hydriermetall in einem heterogenen, stark sauren Katalysator, der ein Hydriermetall aufweist, kombiniert sind.

13. Verfahren nach einem der Ansprüche 1 bis 11, wobei der stark saure Katalysator ein flüssiger, stark saurer Katalysator ist und das Hydriermetall auf einem festen nicht-sauren Katalysatorträger geträgert ist.

14. Verfahren nach einem der vorhergehenden Ansprüche, weiter umfassend den folgenden Schritt:
(g) Gewinnen von Pentansäure als Produkt aus dem mit Pentansäure angereicherten Strom.

## Revendications

1. Procédé pour effectuer la conversion de l'acide lévulinique en acide pentanoïque, comprenant les étapes suivantes :
(a) l'alimentation d'hydrogène et d'une charge de départ comprenant de l'acide lévulinique vers une première zone catalytique comprenant un catalyseur fortement acide et un métal d'hydrogénation ;
(b) la conversion, dans la première zone catalytique, de l'acide lévulinique à une température comprise dans la plage de 100 à 250 °C en gamma-valérolactone, de manière à obtenir un premier effluent comprenant de la gamma-valérolactone ;
(c) l'alimentation d'au moins une partie du premier effluent vers une seconde zone catalytique comprenant un catalyseur fortement acide et un métal d'hydrogénation ; et
(d) la conversion, dans la seconde zone catalytique, de la gamma-valérolactone en acide pentanoïque à une température comprise dans la plage de 200 à 350 °C, de manière à obtenir un second effluent comprenant de l'acide pentanoïque,
dans lequel la température de conversion dans la première zone catalytique est inférieure à la température de conversion dans la seconde zone catalytique, et dans lequel le catalyseur acide et le métal d'hydrogénation dans la première zone catalytique présentent la même composition que le catalyseur acide et le métal d'hydrogénation dans la seconde zone catalytique.

2. Procédé selon la revendication 1, dans lequel la température de conversion dans la première zone catalytique est comprise dans la plage de 125 à 200 °C.

3. Procédé selon la revendication 1 ou 2, dans lequel la température de conversion dans la seconde zone catalytique est comprise dans la plage de 250 à 300 °C.

4. Procédé selon l'une quelconque des revendications précédentes, dans lequel la température de conversion dans la première zone catalytique présente une valeur inférieure de 30 à 150 °C à la température de conversion dans la seconde zone catalytique.

5. Procédé selon l'une quelconque des revendications précédentes, dans lequel la totalité du premier effluent est délivré à la seconde zone catalytique.

6. Procédé selon l'une quelconque des revendications précédentes, dans lequel la première et la seconde zone catalytique sont contenues dans une seule cuve réactionnelle.

7. Procédé selon la revendication 6, dans lequel les première et seconde zones catalytiques représentent la partie en amont et la partie en aval d'un seul lit catalytique, respectivement.

8. Procédé selon l'une quelconque des revendications précédentes, dans lequel le volume de la première zone catalytique se situe dans la plage de 20 à 80 % en volume par rapport au volume combiné des première et seconde zones catalytiques, de préférence, dans la plage de 30 à 60 % en volume.

9. Procédé selon l'une quelconque des revendications précédentes, dans lequel le second effluent comprend en outre de la gamma-valérolactone, le procédé comprenant encore les étapes suivantes consistant à :
(e) scinder le second effluent sous la forme d'un courant enrichi en gamma-valérolactone et d'un courant enrichi en acide pentanoïque ; et
(f) recycler le courant enrichi en gamma-valérolactone dans la première zone catalytique.

10. Procédé selon la revendication 9, dans lequel le rapport molaire de l'acide lévulinique dans la charge de départ à la gamma-valérolactone recyclée dans la première zone réactionnelle se situe dans la plage de 0,05 à 5,0, de préférence, de 0,1 à 2,0, mieux encore, de 0,2 à 0,5.

11. Procédé selon la revendication 9 ou 10, dans lequel le courant enrichi en gamma-valérolactone est refroidi avant d'être recyclé dans la première zone réactionnelle, de préférence, il est refroidi à une température dans la plage de 20 à 200 °C, mieux encore, de 40 à 100 °C.

12. Procédé selon l'une quelconque des revendications précédentes, dans lequel le catalyseur fortement acide et le métal d'hydrogénation sont combinés sous la forme d'un catalyseur hétérogène fortement acide contenant un métal d'hydrogénation.

13. Procédé selon l'une quelconque des revendications 1 à 11, dans lequel le catalyseur fortement acide est un catalyseur fortement acide liquide et le métal d'hydrogénation est supporté sur un support de catalyseur solide non acide.

14. Procédé selon l'une quelconque des revendications précédentes, le procédé comprenant en outre l'étape suivante consistant à :
(g) récupérer l'acide pentanoïque sous la forme d'un produit issu du courant enrichi en acide pentanoïque.
